# EUROPEAN PATENT SPECIFICATION

(11) **EP 3 412 649 B1**
(45) Date of publication and mention of the grant of the patent: **04.03.2020**
(21) Application number: 17746749.5
(22) Date of filing: 16.01.2017
(51) Int. Cl.: C07C 67/14, C07C 69/24, C07C 29/17, C07C 31/135, C07C 229/12, C07D 295/15, C07C 291/02, C07D 211/74, A61K 31/045, A61K 31/22, A61K 31/5375, A61K 31/4453, A61K 31/45, A61P 11/06

(54) **TERPINEOL AND PREPARATION METHOD AND APPLICATION THEREOF**
TERPINEOL UND HERSTELLUNGSVERFAHREN UND ANWENDUNGEN DAVON
TERPINÉOL ET PROCÉDÉ DE PRÉPARATION ET APPLICATION CORRESPONDANTS

(30) Priority: 04.02.2016 CN 201610079970
(43) Date of publication of application: 12.12.2018
(73) Proprietor: Zhejiang Academy Of Traditional Chinese Medicine, Hangzhou, Zhejiang 310007 (CN)
(72) Inventor: ZHU, Wanping, Hangzhou Zhejiang 310007 (CN); HU, Yongzhou, Hangzhou Zhejiang 310007 (CN); LIU, Xia, Hangzhou Zhejiang 310007 (CN); KONG, Fanzhi, Hangzhou Zhejiang 310007 (CN); WANG, Yuji, Hangzhou Zhejiang 310007 (CN)
(74) Representative: Rössler, Matthias
(86) International application number: PCT/CN2017/071217
(87) International publication number: WO 2017/133429

(56) References cited:
- WO-A1-2013/034416
- CN-A- 102 416 154
- CN-A- 103 922 927
- BIAN, RULIAN ET AL.: 'THE PHARMACOLOGICAL PROPERTIES OF A NEW ANTIASTHMATIC DRUG, a-TERPINEOL' CHINESE PHARMACOLOGICAL BULLETIN vol. 3, no. 6, 31 December 1987, ISSN 1001-1978 page 323 AND 326

## Description

### Field of the Invention

The present invention relates to the biomedicine field, in particular to a terpinenol compound and its preparation method and use.

### Background Arts

Blumea oil belongs to a volatile oil as contained in the dry or fresh leaf of Artemisia argyi, which can directly slacken tracheal smooth muscle, and guard against convulsion to the tracheal smooth muscle and asthma as incurred by antigen, acetylcholine and histamine. It also has anti-allergic effect to inhibit allergic slow reacting substance (SRS-A) released by sensitized lung tissue, and guard against SRS-A and 5--hydroxy tryptamine. It is also available for phlegm elimination and cough suppression. It exerts no excitation to the heart, which is mainly used for therapy of bronchial asthma and asthmatic chronic bronchitis.

A-terpinenol is an active ingredient extracted from the Blumea oil for asthma suppression, of which chemical designation is α,α,4-Trimethylcyclohex-3-en-1-methanol. According to pharmacological action of α-terpinenol reported by Bian Rulian and his colleagues ("a new antiasthmatic drug- pharmacological action of α-terpinenol", Chinese Pharmacological Bulletin, Page 323-328, Vol. 3, No.6, 1987.), α-terpinenol is available for air tube (trachea) relaxation during vivo experiment and experiment in vitro to guinea pig, which can increase cAMP content in the tracheal smooth muscle; it also plays a role of anti anaphylaxis, cough suppression and phlegm elimination, which is a safe and effective new antiasthmatic drug owing to its low toxicity. As different from β receptor agonist, α-terpinenol has inhibiting effect on isolated atria of guinea pig.

Chinese Patent Application with the publication number of CN 103922927 A discloses a derivative of α-terpinenol as well as its preparation method and use; structure of such derivative of α-terpinenol is shown as follows:

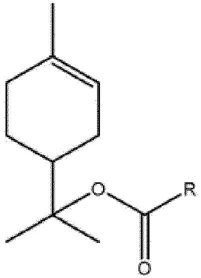

Wherein, R is selected from C₁∼C₅ alkyl; however, the anti-asthmatic activity is not satisfactory despite of the fact that it has been enhanced to some extent through derivatization of α-terpinenol.

WO 2013/034416 relates to a method for disinfection involving an antimicrobial composition, an antimicrobial composition suitable for use in such a method and antimicrobial compounds. It particularly relates to an antimicrobial composition for personal cleaning, oral care or hard surface cleaning applications. It was found that compositions comprising selected terpinyl derivatives and a carrier provide antimicrobial action. In a preferred aspect the compositions of the invention also comprise 1 to 80 %-wt of surfactant.

### Summary of the Invention

The present invention provides an α-terpinenol compound as well as its preparation method and use; such terpinenol compound has higher anti-asthmatic activity and anti-inflammation activity.

An anti-asthmatic compound wtih the structure as shown in Formula (I) or (II): 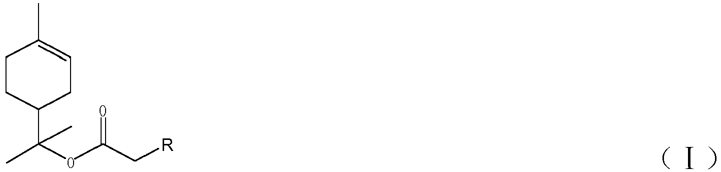 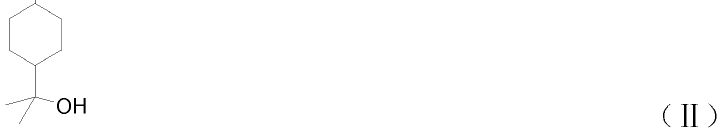
In Formula (I), R is independently selected from C₁₂∼C₁₆ alkyl, -NR¹R², -SR³ or -OR⁴;
R¹, R², R³ and R⁴ are independently selected from C₁∼C₆ alkyl or -NO₂; the said C₁∼C₆ alkyl can be substituted by OH;
Alternatively, the said R¹ and R² form a five-member ring or six-member ring in together with N used to link up them; the said five-member or six-member ring may contain one O or C=O.

As shown by activity test, the terpinenol compound according to the present invention can slacken the tracheal smooth muscle, and reduce the inflammatory cell, which can also effectively alleviate pulmonary hypertension.

In a preferred embodiment, the said R is -NR¹R²; in such case, the said terpinenol compound can form a medically acceptable salt with acids to facilitate dissolution in the water and pharmaceutical use.

In a preferred embodiment, the said R is C₁₂H₂₅, C₁₆H₃₃ or one of the following perssads:

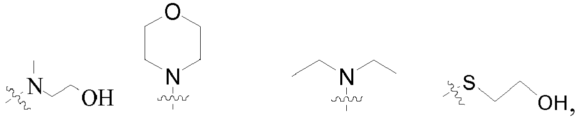

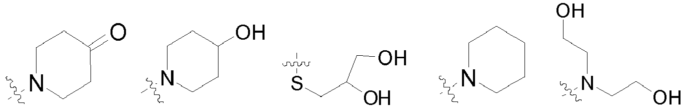

Wherein, " 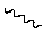 " refers to link location.

Such perssads can ensure higher activity and easy acquisition of compound.

The present invention further provides a method for preparation of the said terpinenol compound.

Where the terpinenol compound is the compound as shown in Formula (I), and R is C₁₂∼C₁₆ alkyl, the preparation method shall comprise the following steps:
Obtain the said terpinenol compound through reaction between the α-terpinenol and alkyl chloride in the dichloromethane under the action of pyridine;
The said alkyl chloride is C₁₄∼C₁₆ alkyl chloride.

Where the terpinenol compound is the compound as shown in Formula (I), and R is -NR¹R², -SR³ or -OR⁴; such preparation method shall comprise the following steps:
(1) Obtain the esterified intermediate through reaction between the α-terpinenol and bromoacetyl bromide in the dichloromethane under the action of pyridine;
(2) Obtain the said terpinenol compound through substitution reaction between the esterified intermediate as obtained in Step (1) and nucleophilic reagent in the acetonitrile under the action of inorganic base;
The said nucleophilic reagent is HNR¹R², HSR³ or HOR⁴.

Where the terpinenol compound is the compound as shown in Formula (II), the preparation method shall comprise the following steps:
Obtain the said terpinenol compound through hydrogenation of α-terpinenol in the methanol in the atmosphere of Pd/C and hydrogen gas;
The present invention further provides an use of the said terpinenol compound in pharmaceutical field.

In a preferred embodiment, the said terpinenol compound is used to prepare anti-asthmatic drug;
The said anti-asthmatic drug is used to relax bronchus.

As indicated by testing results, the said terpinenol compound can also effectively reduce inflammatory cells; in a further preferred embodiment, the said terpinenol compound is used to prepare anti-inflammatory drug.

In still a further preferred embodiment, the said terpinenol compound is used to prepare drugs for therapy or alleviation of pulmonary hypertension.

As compared with prior arts, the present invention has the following beneficial effect:
(1) The present invention has effectively improved the role of such compound in guarding against asthma and pulmonary hypertension through derivatization of terpinenol compound;
(2) The terpinenol compound according to the present invention can reduce inflammatory cells, which has higher anti-inflammation activity.

### Preferred embodiments

### Embodiment 1

Dissolve α-terpinenol (4mmol) as taken by precise weighing in 10ml dichloromethane, and add (6mmol) pyridine as well as myristoyl chloride (6mmol) subjecting to ice bathing for reaction for 8h at room temperature to obtain the final product 2a (3.74mmol, yield rate of 93.5%). Product structure is as follows:

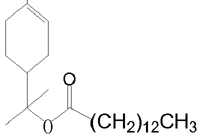

### Embodiment 2

Dissolve α-terpinenol (4mmol) as taken by precise weighing in 10ml dichloromethane, and add (6mmol) pyridine as well as Octadecanoyl chloride (6mmol) subjecting to ice bathing for reaction for 8h at room temperature to obtain the final product 2b (3.86mmol, yield rate of 96.5%).

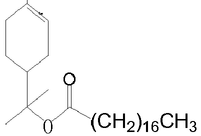

### Embodiment 3∼12

(1) Take 1.85g (10mmol) α-terpinenol by weighing, and put it into 50ml round-bottom flask; further add 30ml dichloromethane, 1.91ml (20mmol) pyridine and 2.09ml (20mmol) bromoacetyl bromide subjecting to ice bathing for reaction at room temperature for 7h to obtain intermediate 3 (5.9mmol, yield rate of 48.2%).
Reaction equation is stated as follows: 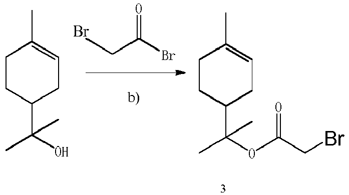
(2) Dissolve intermediate 3 (274mg, 1mmol) in 10mL acetonitrile, and add potassium carbonate (276.4mg, 2mmol) and intermediate 4 (2mmol) at room temperature to obtain product 5; structure and yield rate of intermediate 4 is as shown in Table 1.

**Table 1: Substrate in Embodiment 3-12 and Results**

| Embodiment | Intermediate 3 | Yield | Yield Coefficient |
|---|---|---|---|
| 3 | 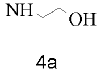 | 0.765mmol | 76.5% |
| 4 | 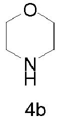 | 0.842mmol | 84.2% |
| 5 | 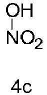 | 0.748mmol | 74.8% |
| 6 | 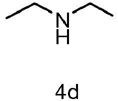 | 0.914mmol | 91.4% |
| 7 | 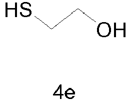 | 0.839mmol | 83.9% |
| 8 | 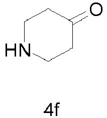 | 0.812mmol | 81.2% |
| 9 | 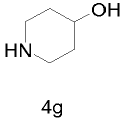 | 0.828mmol | 82.8% |
| 10 | 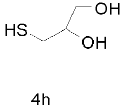 | 0.87mmol | 87% |
| 11 | 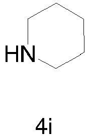 | 0.873mmol | 87.3% |
| 12 | 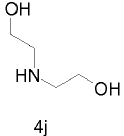 | 0.635mmol | 63.5% |

### Embodiment 13

Add α-terpinenol (400mg) into 10mL methanol, and further add Pd/C (40 mg) for agitation in the hydrogen environment for 8h. Obtain colorless oily substance 6 (38mg, 93.5%) through separation. Reaction equation is as follows:

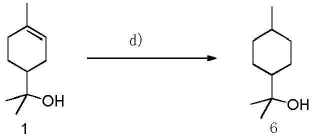

### Data on structural characteristics of partial compounds is as follows: Compound A:

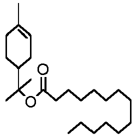

**¹H NMR (500 MHz, CDCl₃):** δ 5.37 (s, 1H), 2.25 - 2.16 (m, 2H), 2.07 - 1.90 (m, 4H), 1.89 - 1.76 (m, 2H), 1.64 (s, 3H), 1.61 - 1.52 (m, 2H), 1.43 (s, 3H), 1.41 (s, 3H), 1.25 (s, 21H), 0.91 - 0.82 (m, 3H).

### Compound B

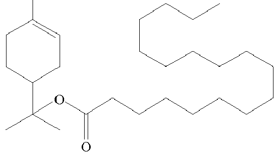

**¹H NMR (500 MHz, CDCl₃):** δ 5.36 (dd, *J* = 8.3, 6.9 Hz, 1H), 2.20 (t, *J* = 7.5 Hz, 2H), 2.08 - 1.91 (m, 4H), 1.88 - 1.75 (m, 2H), 1.64 (s, 3H), 1.58 - 1.53(m, 2H), 1.44 (s, 3H), 1.41 (s, 3H), 1.34 - 1.19 (m, 29H), 0.88 (t, *J* = 7.0 Hz, 3H).

### Compound C

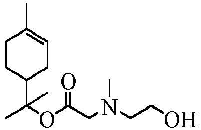

**¹H NMR (500 MHz, CDCl₃):** δ 5.38 (d, *J* = 2.0 Hz, 1H), 3.62 - 3.58 (m, 2H), 3.24 (s, 2H), 2.73 - 2.69 (m, 2H), 2.44 (s, 3H), 2.13 - 1.93 (m, 4H), 1.89 - 1.76 (m, 2H), 1.66 (s, 3H), 1.49 (s, 3H), 1.46 (s, 3H), 1.32 (tt, *J* = 10.4, 5.3 Hz, 1H).

### Compound D

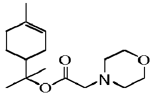

**¹H NMR (500 MHz, CDCl₃):** δ 5.38 (dd, *J* = 3.1, 1.7 Hz, 1H), 3.78 (dd, *J* = 8.3, 3.6 Hz, 4H), 3.13 (d, *J* = 1.3 Hz, 2H), 2.64 - 2.56 (m, 4H), 2.12 - 1.92 (m, 4H), 1.89 - 1.76 (m, 2H), 1.66 (s, 3H), 1.48 (s, 3H), 1.45 (s, 3H), 1.36 - 1.26 (m, 1H).

### Compound F

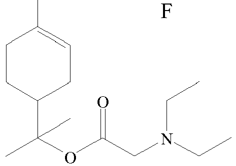

**¹H NMR (500 MHz, CDCl₃):** 5.38 (dd, *J* = 2.9, 1.5 Hz, 1H), 3.23 (s, 2H), 2.67 (q, *J* = 7.3 Hz, 4H), 2.10 - 1.93 (m, 4H), 1.88 - 1.76 (m, 2H), 1.65 (s, 3H), 1.47 (s, 3H), 1.44 (s, 3H), 1.35 - 1.25 (m, 1H), 1.08 (td, *J* = 7.2, 4.1 Hz, 6H).

### Compound G

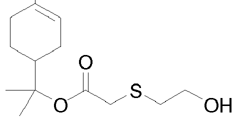

**¹H NMR (500 MHz, CDCl₃):** δ 5.37 - 5.34 (m, 1H), 3.75 (t, *J* = 5.6 Hz, 2H), 3.18 (s, 2H), 2.81 (t, *J* = 5.5 Hz, 2H), 2.09 - 1.94 (m, 4H), 1.86 - 1.76 (m, 2H), 1.63 (s, 3H), 1.45 (s, 3H), 1.43 (s, 3H), 1.35 - 1.25 (m, 1H);

### Compound H

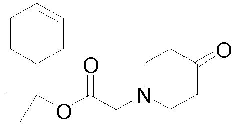

**¹H NMR (500 MHz, CDCl₃):** δ 5.33 (t*, J* = 8.9 Hz, 1H), 3.24 (s, 2H), 2.87 (t, *J* = 6.1 Hz, 4H), 2.48 (t, *J* = 6.1 Hz, 4H), 2.09 - 1.89 (m, 4H), 1.86 - 1.73 (m, 2H), 1.63 (s, 3H), 1.46 (s, 3H), 1.43 (s, 3H), 1.34 - 1.25 (m, 1H);

### Compound J

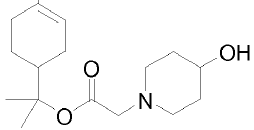

**¹H NMR (500 MHz, CDCl₃):** δ 5.35 (d, *J* = 2.1 Hz, 1H), 3.73 - 3.66 (m, 1H), 3.11 (s, 2H), 2.81 (td, *J* = 8.9, 3.8 Hz, 2H), 2.38 - 2.30 (m, 2H), 2.10 - 1.88 (m, 4H), 1.85 - 1.74 (m, 2H), 1.68 - 1.58 (m, 6H), 1.45 (s, 3H), 1.42 (s, 3H), 1.33 - 1.26 (m, 1H);

### Compound K

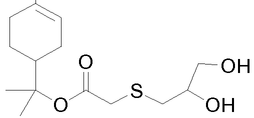

**¹H NMR (500 MHz, CDCl₃):** δ 5.35 (s, 1H), 3.81 (s, 1H), 3.71 (d, *J* = 11.0 Hz, 1H), 3.60 - 3.46 (m, 1H), 3.27 - 3.15 (m, 2H), 2.85 - 2.77 (m, 1H), 2.68 (dd, *J* = 14.0, 8.4 Hz, 1H), 2.09 - 1.90 (m, 4H), 1.86 - 1.75 (m, 2H), 1.63 (s, 3H), 1.45 (s, 3H), 1.43 (s, 3H), 1.33 - 1.25 (m, 1H);

### Compound L

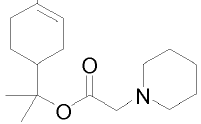

**¹H NMR (500 MHz, CDCl₃):** δ 5.42 - 5.33 (m, 1H), 3.06 (d, *J* = 1.2 Hz, 2H), 2.55 - 2.43 (m, 4H), 2.10 - 1.89 (m, 4H), 1.86 - 1.71 (m, 2H), 1.62 (s, 3H), 1.59 (dd, *J* = 11.3, 5.7 Hz, 4H), 1.43 (d, *J* = 5.8 Hz, 3H), 1.42 - 1.37 (m, 5H), 1.27 (ddd, *J* = 24.4, 12.2, 5.7 Hz, 1H);

### Compound M

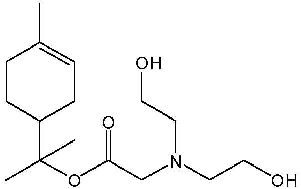

**¹H NMR (500 MHz, CDCl₃):** δ 5.33 (s, 1H), 3.56- 3.54 (m, 1H), 3.11 (s, 2H), 2.81 (td, *J* = 8.9, 3.8 Hz, 2H), 2.38 - 2.30 (m, 2H), 2.10 - 1.88 (m, 4H), 1.85 - 1.74 (m, 2H), 1.68 - 1.58 (m, 6H), 1.45 (s, 3H), 1.42 (s, 3H), 1.33 - 1.26 (m, 1H);

### Compound N

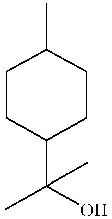

**¹H NMR (500 MHz, CDCl₃):** δ 1.75 (ddd, *J* = 16.6, 10.2, 4.5 Hz, 4H), 1.43 - 1.42 (m, 1H), 1.29 - 1.17 (m, 2H), 1.13 (s, 6H), 1.07 - 0.96 (m, 2H), 0.89 (dd, *J* = 12.0, 2.9 Hz, 1H), 0.85 (d, *J* = 6.5 Hz, 3H).

### Bronchiectasis and anti-inflammation experiment

Method: Fabricate isolated tracheal strip, and place it in the in-vitro tracheal experiment device; add 100µL acetylcholine chloride following tension adjustment and balancing to make the trachea extract, and increase the tension of trachea; once the tension is increased to the maximum value, and becomes balanced, feed corresponding trial drug to inhibit extraction of the trachea; record isometric muscle tension variation curve, and calculate diastolic rate of smooth muscle. Diastolic rate = (tension after radiography-tension after dosing)/(tension after radiography)^{∗}100%.

Establish rat asthma model, and proceed with blunt dissection of trachea following the last feeding of drug for trachea alveolar washing; collect bronchi alveolar lavage fluid (BALF), and count total number of nucleated cells. Results are as shown in Table 2.

**Table 2**

| Group | Relaxation rate % | | | |
|---|---|---|---|---|
| | | 0.75mmol/L | 1.25mmol/L | Inflammatory cell (×10⁸/L) |
| Blank group | R | 7.73±4.81 | 13.59±9.14 | 85.50±18.70^{##} |
| Model group | | | | 182.50±24.52^{ΔΔ} |
| Aminoph ylline | | 43.67±10.20^{ΔΔ} | 56.70±9.26^{ΔΔ} | |
| A^{a} | H | 14.62±4.24** | 21.57±7.04**Δ | |
| B^{a} | CH₃ | 34.35±14.72 | 47.72±15.94 | |
| C^{a} | CH₂CH₃ | 18.18±6.26** | 23.22±4.39**Δ | |
| N^{a} | CH₂CH₂CH₃ | 21.20±9.24** | 25.47±8.69** | |
| ISO^{a} | | 19.88±8.82** | 22.79±8.27**Δ | |
| TR^{a} | 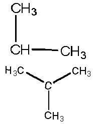 | 25.49±6.53* | 28.02±8.83** | |
| α-T | | 28.40±16.13^{ΔΔ} | 38.35±13.62^{Δ} | 113.00±21.27^{#} |
| A | -(CH₂)₁₂CH₃ | 17.56±2.89**^{ΔΔ} | 16.55±14.47** | 139.50±31.24^{Δ} |
| B | -(CH₂)₁₆CH3 | 15.4±9.83** | 10.63±13.77** | 134.50.25.27^{Δ} |
| D | 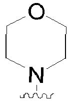 | 36.60±13.79^{ΔΔ} | 59.35±14.66^{Δ} | 110.5±16.21^{##} |
| E | 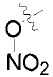 | 17.13±6.98** | 10.84±4.46** | 139.50±20.71^{Δ} |
| F | 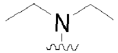 | 42.33±10.45^{ΔΔ} | 46.71±3.56*^{ΔΔ} | 101.50±14.68^{#} |
| G | 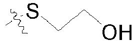 | 48.29±9.36^{ΔΔ} | 81.09±10.15**^{ΔΔ} | 76.00±18.15^{##} |
| H | 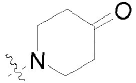 | 20.85±7.55**^{Δ} | 43.81±10.16*^{ΔΔ} | 123.00±17.23^{#} |
| J | 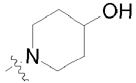 | 35.17±13.87^{ΔΔ} | 71.53±15.45^{ΔΔ} | 99.50±21.23^{##} |
| K | 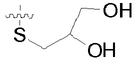 | 34.84±19.74 | 65.29±13.43^{Δ} | 104.50±13.25^{##} |
| L | 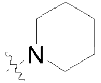 | 42.49±6.14^{ΔΔ} | 52.21±6.98^{Δ} | 110.50±16.18^{##} |
| M | 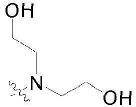 | 27.74±2.08*^{ΔΔ} | 61.01±5.07^{ΔΔ} | 97.50±13.11^{##} |
| N | 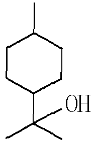 | 2716ci14.10* | 41.28±13.17*^{ΔΔ} | 140.00±26.21^{Δ} |

| | | | | |
|---|---|---|---|---|
| ^{a} represents several compounds in preferred embodiments of CN 103922927 A, formula for computation of diastolic rate has been improved to some extent; meanwhile, as there exists certain difference to testing results of different batches, thus, data on diastolic rate is also varied. " 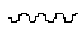 " refers to substitution position; compound N is product structure. ** refers to P<0.01 as compared with α-terpinenol group; *refer to P< 0.05 as compared with α-terpinenol group; ΔΔ refers to P<0.01 as compared with blank group; Δ refers to P< 0.05 as compared with blank group. | | | | |

As compared with model group, # *P*<0.05 , ## *P*<0.01

Various compound groups can increase the diastolic rate of trachea smooth muscle, and inhibit inflammation under different dosage. On this account, it can be used to cure inflammation incurred by asthma, chronic obstructive pulmonary diseases and various other diseases, such as arthritis, rheumatoid arthritis, bronchitis, allergic rhinitis and allergic dermatitis.

### Impact on monocrotaline incurred pulmonary hypertension to rats

Method: Establish rat PAH model, and feed normal saline to contrast group with volume equivalent to that for injection. Feed drug for interference 2 days after radiography; feed 200mg/Kg per compound on daily basis; use normal saline with equivalent volume for lavage to the contrast group and model group respectively. Measure right ventricular systolic pressure of rat by means of Right cardiac catheterization at anesthesia state on the 30^{th} day. Results are as shown in Table 3.

**Table 3: Right Ventricular Systolic Pressure of Different Groups (x̅ ± S)**

| | n | RVSP (mmHg) |
|---|---|---|
| Normal group | 10 | 21.08±2.13^{Δ#} |
| Model group | 8 | 43.78±2.14^{#} |
| α-terpmeno 1 group | 8 | 33.03±2.22^{Δ} |
| A | 8 | 35.07±2.81 |
| B | 10 | 36.08±2.01 |
| D | 8 | 32.04±1.12^{Δ} |
| E | 8 | 35.53±3.02 |
| F | 8 | 23.35±1.04^{ΔΔ##} |
| G | 8 | 21.08±1.31^{ΔΔ#} |
| H | 8 | 30.33±2.03^{Δ} |
| J | 8 | 27.06±1.17^{Δ} |
| K | 8 | 31.13±2.03^{Δ} |
| L | 8 | 25.36±1.17^{ΔΔ#} |
| M | 8 | 33.36±2.05^{Δ} |
| N | 8 | 32.56±2.82^{Δ} |

As compared with model group, Δ *P*<0.05, ΔΔP<0.01; as compared with α-terpinenol group, # *P<0.05* , ## *P<*0.01

As indicated by testing results, various compounds can provide certain protection for monocrotaline incurred rat pulmonary artery hypertension; furthermore, they can improve hemodynamic indicators, reduce right ventricular systolic pressure, alleviate right ventricular load, reduce Right heart hypertrophy indicators, and alleviate pulmonary vascular remodeling.

## Claims

1. A terpinenol compound, **characterized in that** its structure is as shown in Formula (I): 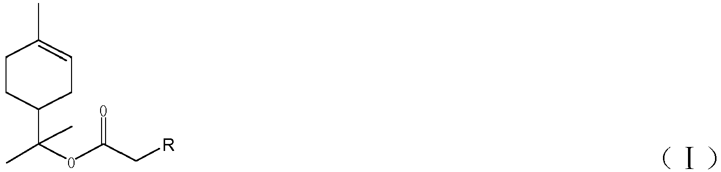 The said R is one of the following perssads: 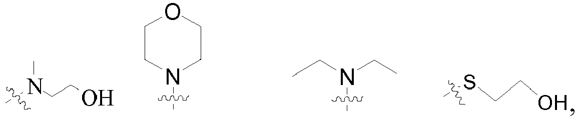 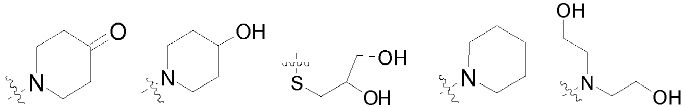 Wherein, " 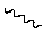 " refers to link location.

2. A method for preparation of terpinenol compound according to Claim 1, **characterized in that** it comprises the following steps:
(1) Obtain the esterified intermediate through reaction between the α-terpinenol and bromoacetyl bromide in the dichloromethane under the action of pyridine;
(2) Obtain the said terpinenol compound through substitution reaction between the esterified intermediate as obtained in Step (1) and nucleophilic reagent in the acetonitrile under the action of inorganic base;
The said nucleophilic reagent is in one of the following structural equations:

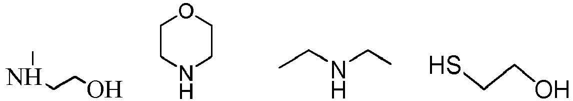

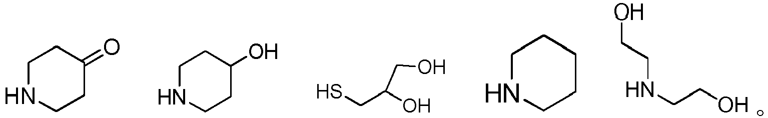

3. Terpinenol compound according to Claim 1 for use in the pharmaceutical field, **characterized in that** the said terpinenol compound is for use as an anti-asthma drug.

4. Terpinenol compound for use according to Claim 3, wherein the compound is for use in the treatment of bronchial dilation.

5. Terpinenol compound according to Claim 1 for use in the pharmaceutical field, **characterized in that** the said terpinenol compound is for use as an anti-inflammatory drug.

6. Terpinenol compound according to Claim 1 for use in the pharmaceutical field, **characterized in that** the said terpinenol compound is for use in the therapy or alleviation of pulmonary hypertension.

## Patentansprüche

1. Eine Terpineolverbindung, **dadurch gekennzeichnet, dass** ihre Struktur wie in Formel (I) dargestellt ist: 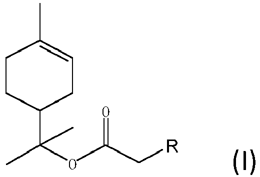 Der genannte R ist einer der folgenden Resten: 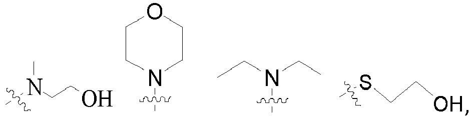 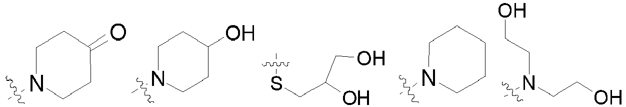 wobei " 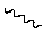 " die Stelle der Bindung bezeichnet.

2. Ein Verfahren zur Herstellung der Terpinenolverbindung nach Anspruch 1, **dadurch gekennzeichnet, dass** es die folgenden Schritte umfasst:
(1) Erhalten des veresterten Zwischenprodukts durch Reaktion zwischen α-Terpinenol und Bromacetylbromid in Dichlormethan unter Einwirkung von Pyridin;
(2) Erhalten der genannten Terpinenolverbindung durch Substitutionsreaktion zwischen dem in Schritt (1) erhaltenen veresterten Zwischenprodukt und dem nucleophilen Reagenz in Acetonitril unter Einwirkung einer anorganischen Base;
Das nucleophile Reagenz ist eines der folgenden Strukturformeln:

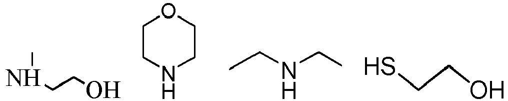

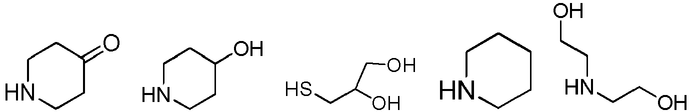

3. Die Terpinenolverbindung nach Anspruch 1 zur Verwendung im pharmazeutischen Bereich, **dadurch gekennzeichnet, dass** die genannte Terpinenolverbindung zur Verwendung als Antiasthmatikum bestimmt ist.

4. Die Terpinenolverbindung zur Verwendung nach Anspruch 3, wobei die Verbindung zur Verwendung bei der Behandlung von Bronchodilatation bestimmt ist.

5. Die Terpinenolverbindung nach Anspruch 1 zur Verwendung im pharmazeutischen Bereich, **dadurch gekennzeichnet, dass** die genannte Terpinenolverbindung zur Verwendung als entzündungshemmendes Mittel bestimmt ist.

6. Die Terpinenolverbindung nach Anspruch 1 zur Verwendung im pharmazeutischen Bereich, **dadurch gekennzeichnet, dass** die genannte Terpinenolverbindung zur Verwendung in der Therapie oder zur Linderung der pulmonalen Hypertonie bestimmt ist.

## Revendications

1. Composé de terpinénol, **caractérisé en ce que** sa structure est telle que représentée dans la Formule (I): 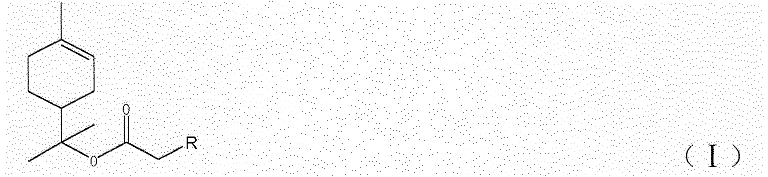 ledit R est l'une des fonctions suivantes: 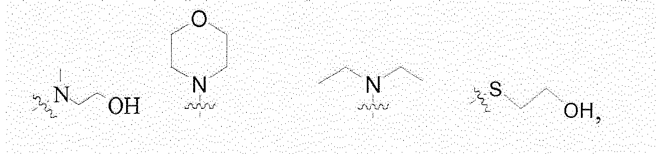 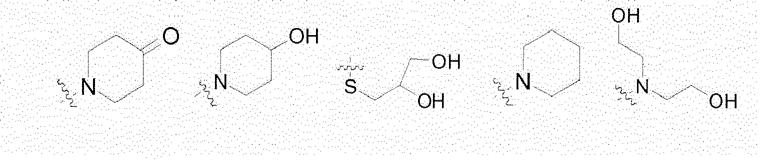 dans lequel « 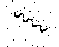 » désigne l'emplacement du lien.

2. Procédé de synthèse d'un composé de terpinénol selon la Revendication 1, **caractérisé en ce qu'**il comprend les étapes suivantes:
(1) obtention de l'intermédiaire estérifié par réaction entre le α-terpinénol et le bromure de bromoacétyle dans le dichlorométhane sous l'action de la pyridine;
(2) obtention dudit composé de terpinénol par réaction de substitution entre l'intermédiaire estérifié tel qu'obtenu dans l'étape (1) et le réactif nucléophile dans l'acétonitrile sous l'action d'une base inorganique;
ledit réactif nucléophile est dans l'une des équations structurales suivantes:

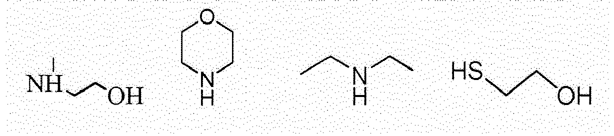

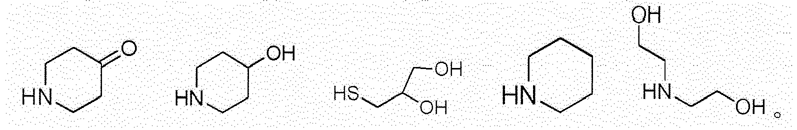

3. Composé de terpinénol selon la Revendication 1 pour utilisation dans le domaine pharmaceutique, **caractérisé en ce que** ledit composé de terpinénol est destiné à une utilisation en tant que médicament anti-asthme.

4. Composé de terpinénol pour utilisation selon la Revendication 3, dans lequel le composé est destiné à une utilisation dans le traitement de la dilatation des bronches.

5. Composé de terpinénol selon la Revendication 1 pour utilisation dans le domaine pharmaceutique, **caractérisé en ce que** ledit composé de terpinénol est destiné à une utilisation en tant que médicament anti-inflammatoire.

6. Composé de terpinénol selon la Revendication 1 pour utilisation dans le domaine pharmaceutique, **caractérisé en ce que** ledit composé de terpinénol pour utilisation dans le traitement ou le soulagement de l'hypertension pulmonaire.
